# EUROPEAN PATENT SPECIFICATION

(11) **EP 1 562 577 B1**
(45) Date of publication and mention of the grant of the patent: **05.10.2011**
(21) Application number: 03775788.7
(22) Date of filing: 23.10.2003
(51) Int. Cl.: A61K 31/221, A61K 31/454, A61P 25/02

(54) **ACETYL-L-CARNITINE FOR THE PREVENTION AND/OR TREATMENT OF PERIPHERAL NEUROPATHIES INDUCED BY THALIDOMIDE**
ACETYL-L-CARNITIN FÜR DIE VORBEUGUNG UND/ODER BEHANDLUNG VON PERIPHEREN NEUROPATHIEN HERVORGERUFEN DURCH THALIDOMID
ACETYLE-L-CARNITINE POUR LA PREVENTION ET/OU LE TRAITEMENT DE NEUROPATHIES PERIPHERIQUES INDUITES PAR LA THALIDOMIDE

(30) Priority: 13.11.2002 US 292823
(43) Date of publication of application: 17.08.2005
(73) Proprietor: SIGMA-TAU Industrie Farmaceutiche Riunite S.p.A., 00144 Roma (IT)
(72) Inventor: CAVAZZA, C., c/o Sigma-Tau Ind. Farma. Riunite SPA, I-00040 Pomezia RM (IT); PISANO, C., c/o Sigma-Tau Ind. Farma. Riunite SPA, I-00040 Pomezia RM (IT); VESCI, L., c/oSigma-Tau Ind. Farma. Riunite SPA, I-00040 Pomezia RM (IT)
(74) Representative: Spadaro, Marco
(86) International application number: PCT/IT2003/000656
(87) International publication number: WO 2004/043454

(56) References cited:
- US-A- 4 751 242
- US-A1- 2001 044 465
- STASI M A ET AL: "Neuroprotective effect of acetyl-l-carnitine (ALCAR) on peripheral neuropathy induced by taxol and cisplatin" FASEB JOURNAL, vol. 14, no. 4, 15 March 2000 (2000-03-15), page A632 XP009027698 Annual Meeting of Professional Research Scientists: Experimental Biology 2000;San Diego, California, USA; April 15-18, 2000 ISSN: 0892-6638
- VITALI G ET AL: "Symptomatic and neurophysiological response of paclitaxel and/or cisplatin neuropathy to oral acetyl-L-carnitine (ALCAR)" ANNALS OF ONCOLOGY, vol. 13 (suppl.1), 2002, page 179 (659P) XP009027696 cited in the application
- PISANO CLAUDIO ET AL: "Acetyl-L-carnitine protects from peripheral neuropathy and reduces bone marrow injury in Taxol-treated mice" PROCEEDINGS OF THE AMERICAN ASSOCIATION FOR CANCER RESEARCH ANNUAL, no. 41, March 2000 (2000-03), pages 607-608, XP001180236 91st Annual Meeting of the American Association for Cancer Research.;San Francisco, California, USA; April 01-05, 2000, March, 2000 ISSN: 0197-016X
- CAVALETTI G ET AL: "Relationship between acetyl-L-carnitine and nerve growth factor: results of in vitro and in vivo neuroprotection studies" JOURNAL OF NEUROLOGY, vol. 249 (suppl.1), June 2002 (2002-06), page 1/28 (78) XP009027808 cited in the application
- MAESTRI ET AL: "Acetyl-L-carnitine (ALCAR) in patients with chemotherapy-induced peripheral sensory neuropathy" PROCEEDINGS OF THE AMERICAN SOCIETY OF CLINICAL ONCOLOGY, vol. 21, 2002, page 247b (2807) XP009027807 & CAVALETTI G ET AL: "Current status and future prospects for the treatment of chemotherapy-induced peripheral neurotoxicity" EUROPEAN JOURNAL OF CANCER, PERGAMON PRESS, OXFORD, GB, vol. 38, no. 14, September 2002 (2002-09), pages 1832-1837, XP004377449 ISSN: 0959-8049
- LUDUENA R F ET AL: "INTERACTIONS OF VINBLASTINE AND MAYTANSINE WITH TUBULIN", SOIFER, D. (ED.). ANNALS OF THE NEW YORK ACADEMY OF SCIENCES, VOL. 466. DYNAMIC ASPECTS OF MICROTUBULE BIOLOGY; CONFERENCE, NEW YORK, N.Y., USA, DEC. 3-6, 1984. XIV+978P. THE NEW YORK ACADEMY OF SCIENCES: NEW YORK, N.Y., USA. ILLUS SERIES : ANNALS OF, 1986, pages 718-732, ISSN: null
- GROLLEAU FRANCOISE ET AL: "A possible explanation for a neurotoxic effect of the anticancer agent oxaliplatin on neuronal voltage-gated sodium channels", JOURNAL OF NEUROPHYSIOLOGY (BETHESDA), vol. 85, no. 5, May 2001 (2001-05), pages 2293-2297, ISSN: 0022-3077
- DE SANTIS S ET AL: "Patients treated with antitumor drugs displaying neurological deficits are characterized by a low circulating level of nerve growth factor.", CLINICAL CANCER RESEARCH : AN OFFICIAL JOURNAL OF THE AMERICAN ASSOCIATION FOR CANCER RESEARCH JAN 2000 LNKD- PUBMED:10656436, vol. 6, no. 1, January 2000 (2000-01), pages 90-95, ISSN: 1078-0432
- APFEL STUART C: "Neurotrophic factors in peripheral neuropathies: Therapeutic implications", BRAIN PATHOLOGY, vol. 9, no. 2, April 1999 (1999-04), pages 393-413, ISSN: 1015-6305
- APFEL STUART C ET AL: "Efficacy and safety of recombinant human nerve growth factor in patients with diabetic polyneuropathy: A randomized controlled trial", JAMA (JOURNAL OF THE AMERICAN MEDICAL ASSOCIATION), vol. 284, no. 17, 1 November 2000 (2000-11-01), pages 2215-2221, ISSN: 0098-7484
- ALOE L ET AL: "Evidence that nerve growth factor promotes the recovery of peripheral neuropathy induced in mice by Cisplatin: Behavioral, structural and biochemical analysis", AUTONOMIC NEUROSCIENCE BASIC AND CLINICAL, vol. 86, no. 1-2, 20 December 2000 (2000-12-20), pages 84-93, ISSN: 1566-0702
- CAVALETTI G ET AL: "Circulating nerve growth factor level changes during oxaliplatin treatment-induced neurotoxicity in the rat.", ANTICANCER RESEARCH, vol. 22, no. 6C, November 2002 (2002-11), pages 4199-4204, ISSN: 0250-7005
- HAYAKAWA KAZUHIRO ET AL: "NGF prevention of neurotoxicity induced by cisplatin, vincristine and taxol depends on toxicity of each drug and NGF treatment schedule: In vitro study of adult rat sympathetic ganglion explants", BRAIN RESEARCH, vol. 794, no. 2, 1 June 1998 (1998-06-01), pages 313-319, ISSN: 0006-8993
- TREDICI GIOVANNI ET AL: "Effect of recombinant human nerve growth factor on cisplatin neurotoxicity in rats", EXPERIMENTAL NEUROLOGY, vol. 159, no. 2, October 1999 (1999-10), pages 551-558, ISSN: 0014-4886

## Description

The invention described herein relates to the use of acetyl L-carnitine in the preparation of medicaments useful in the treatment of tumours, particularly in combination with thalichomide

### Background to the invention

It is well-known that the use of anticancer agents in human therapy causes a large number of toxic or side effects which may be life-threatening for the patients. These complications, in fact, may lead to a reduction in the doses of the agents, and occasionally to discontinuation of the therapy itself.

Reduction of the dose or discontinuation of the therapy in many cases causes a deterioration of the individual's general condition because it favours the development of relapses, with consequences which are sometimes fatal for the patient.

Another very important and strongly felt aspect in the hospital setting and among the families of oncological patients is the concept of "improving the quality of life" of the patients under treatment.

It is equally well known that patients undergoing regular polychemotherapy for cancer are subject to a substantial weight loss.

The growing number and importance of the anticancer agents used in human therapy, the main limitation of which continues to be the occurrence of toxic or side effects, mean that this problem is still a matter for considerable concern.

Thus, the discovery of new agents or new, appropriate combinations of different agents capable of substantially reducing the toxic or side effects caused by anticancer agents used in human therapy is much to be desired.

Previous uses of L-carnitine in combination with anticancer agents are already known.

In experimental animal models, it has been demonstrated that rats treated with doxorubicin alone show a greater weight loss than a group of rats treated with the same substance in combination with L-carnitine (Senekowitsch R, Lohninger A, Kriegel H., Staniek H., Krieglsteiner HP., Kaiser E. Protective effects of carnitine on adriamycin toxicity to heart. In: Kaiser E., Lohninger A., (eds.). Carnitine: its role in lung and heart disorders: 126-137. Karger, Basel-New York, 1987)*.*

US Patent N° 4,713,370 describes the use of carnitine in combination with cytostatic agents such as daunomycin, N-acetyl-daunomycin and daunomycin oxime to reduce the cardiac toxicity of these compounds. US Patent N° 4,267,163 describes the use of carnitine in combination with cytostatic agents such as adriamycin, adriamycin-14-octanoate, 4'-epi-adriamycin, adriamycin beta-anomer and 4'-epi-adriamycin gamma-anomer to reduce the cardiac toxicity of these compounds. US patent N° 4,751,242 describes the use of acetyl L-carnitine for the therapeutic treatment of peripheral neuropathies. In this latter patent, peripheral neuropathies are defined as a group of persistent disturbances of the motor neurons of the brain stem and spinal cord and/or the primary sensory neurons and/or the peripheral autonomic neurons, with involvement of the peripheral axons and their attendant supporting structures. With regard to their etiology, peripheral neuropathies constitute a heterogeneous class of diseases because their etiology may be secondary to viral infections (herpes zoster), ischaemia (arteriosclerosis), metabolic unbalances (diabetes, renal and liver insufficiency), drug-induced toxicity (adriamicine, isoniazide, nitrofurantoin), mechanical stresses (compression, entrapment, fracture or dislocation of bones), radiations, genetic factors and pathologies of the immune system. However, no matter what the actual etiological cause of the disease form, is, it is always possible to detect an alteration in the membrane fluidity resulting from an alteration of the cell lipids, cholesterol and gangliosides. Lipids play a very important role in defining the tertiary and quaternary protein structure and in maintaining the stability of adenosin-triphosphatase structure. In fact, their absence brings about the enzyme inactivity. In that reference, it is postulated that acetyl L-carnitine is likely to perform a "scavenger" effect on the free radicals (superoxide, hydroperoxide) that form in conditions of irregular or insufficient perfusion or during phlogistic processes because of the decreased activity of cytochrome oxidase that controls the metabolic shift between the tetravalent and the bivalent anaerobic route. Acetyl L-carnitine, by increasing the levels of reduced glutathione, one of the most important antioxidant metabolites, and probably the levels of the cytochrome oxidases, might bring about the free radicals scavenge and the restoration of the tetravalent oxido-reductant respiratory mechanism in general and of the neurons in particular. Moreover, acetyl L-carnitine would affect the neurons by bringing about an increase in ATP-ase and AChE that are indispensable for optimum neurotransmission.

Other studies have addressed the evaluation of the protective effects of carnitines on anthracycline-induced cardiac toxicity (Neri B., Comparini T., Milani A., Torcia M., Clin. Trial J. 20, 98-103,1983*;* De Leonardis V., De Scalzi M., Neri B., et al. Int. J. Clin. Pharm. Res. 70, 307-311, 1987).

The patent and bibliographical references cited above demonstrate that many efforts have been made in an attempt to reduce the toxic or side effects of anticancer agents, without, however, solving this serious problem in a satisfactory manner.

Carboplatin is a structural analogue of Cisplatin and its associated nephrotoxicity, though by no means negligible, is less than that of Cisplatin. Peripheral neurotoxicity is the dose-limiting side effect of the antineoplastic drug Cisplatin (Cavaletti et al. Journal of the Peripheral Nervous System, Vol. 6, Number 3; September 2001, 136 - 2001 Meeting of the Peripheral Nerve Society).

Vincristine is a well-known anticancer agent which has toxic effects, particularly at the level of the immune system.

Taxol is a natural extract, first isolated from the bark of Taxus brevifolia, with anticancer properties and has proved neurotoxic and myelotoxic in human subjects. It is used for the treatment of tumours resistant to platinum therapy, but gives rise to greater cumulative toxicity in the peripheral nervous system. It has also been ascertained that Taxol induces neutropenia in the subjects treated (Rowinsky e al.; Semin. Oncol. (1993), Aug. 20 (4 suppl 3), 1-15*;* Onetto e al. J. Natl. Cancer Inst. Monogr. (1993); (15):131-9).

Different classes of chemotherapeutic drugs induce peripheral neurotoxicity, among which we mention: taxoids, platinum compounds, vinca alkaloids, the epothilone class, farnesyl transferase inhibitors, thalidomide, 5-fluorouracil, cryptophycin analogues, proteasome inhibitors.

Severe peripheral neuropathy can induce therapy modification, while mild or moderate cases still represent an important tolerability problem limiting patient quality of life.

Whereas the vinca alkaloids bind to tubulin and prevent the polymerisation from soluble dimers into microtubules, the taxoids promote the formation of microtubules and prevent their depolymerisation, which results in an abundance of rigid microtubules.

Microtubules are essential components for the maintenance of cell shape, a variety of cellular actions and axoplasmic transport. Defective function of microtubules in neurons and axons may be the origin of the neurotoxicity of both families of chemotherapeutic agents.

The taxoids induce primarily a symmetrically distributed sensory distal neuropathy, which is related to both single and cumulative doses of the drug and is possibly dependent on the regimen. The neurotoxicity of Taxol is due to a unique mechanism of action; it binds to tubulin and promotes microtubule assembly (Schiff P.B. et al., Nature 1979*;* Parnass J. and Horowitz S.B., J. Cell. Biol. 1981). Microtubules formed in the presence of Taxol are stable in conditions which ordinarily depolymerize them, including the presence of calcium and cold. Paclitaxel also alters the kinetics of microtubule assembly, eliminating the 3- to 4-minute lag time normally observed prior the initiation of assembly (Horowitz S.B. et al., Ann. N.Y. Acad. Sci. 1986). This mechanism contrasts with that of the vinca alkaloids which inhibit microtubule assembly.

This contrasting mechanism makes difficult to find a drug acting at the same time on the two differently induced neuropathy.

With the use of hemopoietic growth factor rescue of myelotoxicity, neurotoxicity becomes the dose-limiting factor (Schiller et al., J. Clin. Oncol. 1994*).* Symptoms may begin as early as 24 to 72 hours after treatment with high single doses (>250 mg/m²), but the neurotoxicity is typically cumulative, with symptoms progressing after each treatment at both high and low doses.

The symptoms are generally tolerable (NCI grade 1 or 2). The neurologic examination characteristically reveals an elevated threshold and perception of vibration in a distal, symmetric, glove-and-stocking pattern. In general, large-fiber modalities (vibration, proprioception) are more frequently affected than loss of small-fiber modalities (pain, temperature). Deep tendon reflexes are also frequently affected, with the distal (ankle) reflex being invariably absent or reduced.

Nerve conduction studies show reductions of sensory nerve action potential amplitudes in a symmetric, distal, length-dependent fashion. Sural sensory nerve action amplitude is virtually always reduced or absent in symptomatic patients.

Mild sensory symptoms have usually improved or resolved completely within several months after discontinuation of Taxol.

Cisplatin induces a peripheral sensory axonal neuropathy, affecting large-diameter and, to a lesser extent, small-diameter sensory fibres. Paraesthesias and impaired propiocepsis are the most common symptoms (Thompson S.W et al., Cancer 1984). Cisplatin accumulates in and damages the dorsal root ganglia, axonal changes being secondary to neuronal damage (Warner E., Int. J. Gynecol. Cancer 1995).

The incidence of Cisplatin-induced neuropathy depends largely on the cumulative dose, the onset of symptoms being seen at a total dose of Cisplatin 330 to 400 mg/m². Cisplatin binds tightly and irreversibly to nerve tissue, which explains the deterioration of neurological condition which sometimes occurs after cessation of Cisplatin therapy.

The vinca alkaloids induce a peripheral sensory-motor polyneurophaty and autonomic neuropathy, which appears to be partially reversible after some months (Potsma T.J. et al., J. Neurooncol. 1993).

The epothilone family has a toxicological profile similar to the taxanes one (Lee FY et al Proc Am Assoc Cancer Res, 2002, 792).

Several other compounds used in cancer treatment show mild to severe neuropathy. Among others, particular mention is made for the farnesyl transferase inhibitors, such as R11577 *(*Adjei, A.A., et al., J. Clin. Oncol., 2003, May, 1; 21 (9): 1760-6); Thalidomide (Rajkumar, S.V., et al., Leukemia, 2003, Apr; 17 (4): 775-9); 5-fluorouracyl (van Laarhoven, H.W., et al., Anticancer Res., 2003, Jan-Feb; 231 (1B): 647-8); Docetaxel (Thongprasert, S., et al., J. Med. Assoc. Thai, 2002, Dec; 85 (12): 1296-300); cryptophycin analogues, such as LY355703 (Sessa, C., et al., Eur. J. Cancer., 2002, Dec; 38 (18): 2388-96) and proteasome inhibitors, such as PS431 (Aghajanian, C., et al., Clin. Cancer Res., 2002, Aug; 8 (8): 2505-11).

One of the general problems of pharmacological therapy is the therapeutic index of the agents, that is to say the ratio of the therapeutically effective dose to the toxic dose, or, at any rate, the dose that gives rise to the onset of side effects.

The medical community still perceives the need for therapeutic regimens which allow the patient to face up to the treatment, which, in the case of anticancer chemotherapy is particularly hard to support, while at the same time conserving an acceptable quality of life. These considerations also apply to the therapeutic treatment of animals, for instance, so-called pets.

The natural tendency to reduce the doses, and thus the use of pharmaceutical forms suitable for therapeutically useful administrations without obliging the patient to take the agents too often, contrasts with the minimum effective doses typical of each anticancer agent.

International application WO 00/06134 discloses the use of L-carnitine and its alkanoyl derivatives in the preparation of medicaments with anticancer agents. In particular, the application discloses the use of combinations of alkanoyl L-carnitine and anticancer agents with an improvement in the therapeutic index and a reduction of the side effects typical of anticancer chemotherapy. In particular, the application discloses the effect of L-carnitine on the general toxicity, expressed as survival time or weight loss for an anticancer agent. More in detail, the cited application provide a synergistic effect by propionyl L-carnitine on anticancer activity, for example of taxol. As far as the specific toxicity of the several anticancer agents is concerned, the cited application provides examples of protective effect against pulmonary toxicity of bleomycin, neutropenia induced by taxol, and taxol-induced peripheral neuropathy.

This general disclosure on the carnitine family would not have led the skilled person to foresee the specific activity of acetyl L-carnitine in the prevention and/or treatment of peripheral neuropathy induced by some anticancer agents.

The same reasoning can be applied in the case of the description of the effects of acetyl L-carnitine in the prevention and/or treatment of peripheral neuropathy induced by cisplatin (Vitali, G. et al., Annals of Oncology, Vol. 13, 2002, Supplement 5: 179, 659P*;* Cavaletti, G. et al., Journal of Neurology, Vol. 249, Suppl. 1, June 2002: 1/28, 78*;* Cavaletti, G. and Zanna, C., European Journal of Cancer, 38 (2002): 1832-7*;* Maestre et al., Proceedings of ASCO, 2002(21), 247; 2807)

### Summary of the invention

It has now been found that the co-ordinated use - this term will be precisely defined below - of thalidomide and acetyl L-carnitine exerts a preventive and/or treating effect on the neuropathy-inducing activity of this anticancer agent.

In the context of the invention described herein, the co-ordinated use of a therapeutically effective amount of thalidomide, and a detoxifying amount of acetyl L-carnitine, or one of its pharmacologically acceptable salts, affords a potent protective effect against the peripheral nerve toxicity and side effects of the anticancer agent, without impairing its efficacy, thus giving rise, amongst other things, to a substantial improvement in the quality of life and a prolonging of life itself in the subjects treated, whether human subjects or animals.

It is an object of the present invention the use of acetyl L-carnitine or of a pharmaceutically acceptable salt thereof for the preparation of a medicament for preventing and/or treating peripheral neuropathies induced by thalidomide said medicament to be administered in a co-ordinated manner to a subject suffering from said peripheral neuropathies, or expected to suffer from said peripheral neuropathies.

Another object of the invention described herein are combinations of acetyl L-carnitine or a pharmaceutically acceptable salt thereof with thalidomide and the related pharmaceutical compositions.

The well known lack of toxic or side effects of acetyl L-carnitine makes the use of this compound particularly safe even for long periods of administration.

The implementation of the invention described herein also contributes to improving the quality of life of the patients treated; one need only think of the physical suffering caused by peripheral neuropathy.

These and other objects of the invention described herein will be described in detail in the embodiment forms of the invention, also by means of examples.

In the context of the invention described herein, the terms "antineoplastic", "anticancer" and "antiproliferative" are to be understood as being essentially synonymous.

### Detailed description of the invention

In the context of the invention described herein, what is meant by "co-ordinated use" of the aforesaid compounds is, indifferently, either (i) co-administration, i.e. the substantially simultaneous or sequential administration of acetyl L-carnitine or one of its pharmacologically acceptable salts and of thalidomide, or (ii) the administration of a composition comprising the aforesaid active ingredients in combination and in a-mixture, in addition to optional pharmaceutically acceptable excipients and/or vehicles.

The invention described herein thus covers both the co-administration of acetyl L-carnitine or one of its pharmacologically acceptable salts and of thalidomide, and pharmaceutical compositions, which can be administered orally, parenterally or nasally, including controlled-release forms, comprising the two active ingredients in a mixture.

The intended use of the co-administration in the context of the present invention means the possibility to perform a preventive treatment (intended also as prevention) for the possible adverse effect, i.e. peripheral neuropathy, of the above anticancer agent. In another embodiment, by the intended use of co-administration, the present invention means the therapeutic treatment (intended also as treatment or therapy) of the adverse effect of this anticancer agent. In the case of preventive treatment, acetyl L-carnitine, shall be administered in view of the need of a treatment with thalidomide, known to imply peripheral neuropathy. The start of the administration, the subsequent schedule (namely, the posology) and the decision upon continuing the administration even after the start of the treatment with this anticancer agent shall be within the knowledge of the clinical expert, depending on the type and severity of the foreseen adverse effect, the conditions of the patient. Just for sake of example, the administration of acetyl L-carnitine can be started immediately before or immediately after surgical removal of the tumor, but in any case before the start of the administration of thalidomide, also in the case this agent be administered before surgery. In case the administration of this anticancer agent is foreseen before, or alternatively to, surgery, both prevention and treatment may apply, whenever the case.

Co-administration also means a package, or manufactured article, comprising distinct administration forms of acetyl L-carnitine or one of its pharmacologically acceptable salts and of thalidomide, accompanied by instructions for the co-ordinated simultaneous or time-scheduled intake of the active ingredients according to a dosage regimen established by the primary care physician, on the basis of the patient's condition. This package is suitable both for prevention and treatment. In a different embodiment of the present invention, the package or manufactured article comprises a unit dosage containing both thalidomide and acetyl L-carnitine.

What is meant by a pharmacologically acceptable salt of acetyl L-carnitine is any salt of the latter with an acid that does not give rise to toxic or side effects. These acids are well known to pharmacologists and to experts in pharmaceutical technology.

Examples of pharmacologically acceptable salts of L-carnitine or of the alkanoyl L-carnitines, though not exclusively these, are chloride; bromide; iodide; aspartate; acid aspartate; citrate; acid citrate; tartrate; acid tartrate; phosphate; acid phosphate; fumarate; acid fumarate; glycerophosphate; glucose phosphate; lactate; maleate; acid maleate; mucate; orotate, oxalate; acid oxalate; sulphate; acid sulphate; trichloroacetate; trifluoroacetate; methane sulphonate; pamoate and acid pamoate. Said salts are disclosed in several patents in the name of the applicant.

One preferred form of daily dosing of acetyl L-carnitine for clinical use is a composition comprising an amount of acetyl L-carnitine from 0.1 to 3 g, and preferably 0.5 to 3 g, or an equivalent amount of a pharmaceutically acceptable salt.

The invention described herein is advantageous in the prevention or treatment of peripheral neuropathy.

What is meant by substantially protective effect is the prevention, reduction or elimination or anyway treatment of the side effect to a statistically significant extent.

The embodiment of the invention described herein also contributes to healing and to prolonging the lives of the patients thanks to the increase in therapeutic success due to the possibility of maintaining the scheduled treatment protocols or of increasing the doses of the chemotherapeutic agent, without having to discontinue the treatment due to contraindications.

A further benefit which is obtained with the invention described herein is related to the quality of life of the subjects treated; in fact, as already mentioned, the elimination or reduction of the physical suffering caused by a peripheral neuropathy favours the patient's ability to be self-sufficient. From the economic standpoint, there are obvious savings in terms of the costs borne by hospital facilities or by the families for the patient's care.

In a first preferred embodiment of the invention described herein, acetyl L-carnitine is administered in a co-ordinated manner with thalidomide.

In a second preferred embodiment of the invention described herein, acetyl L-carnitine has shown an unexpected degree of protective activity against peripheral neuropathy induced by thalidomide. This protective effect shall be understood both as prevention of said side effect and as treatment of said protective effect.

Although the present inventors do not wish to be bound to theoretical speculations, it shall be underlined how the said protective activity (both preventive and therapeutic) by acetyl L-carnitine appeared totally unexpected on the basis of the knowledge in the art. As discussed in the Background section, it is postulated that acetyl L-carnitine is likely to perform a "scavenger" effect on the free radicals by increasing the levels of reduced glutathione. In that section there are also explained the mechanisms by which the anticancer drug of interest in the present invention damage the peripheral nerve. In the case of platin family, it is also well-known that radical scavenger agents are likely to affect drug activity, since if acts through a radicalic mechanism, so the use of a radical scavenger is not advisable.

Acetyl L-carnitine (ALC) exerts its protective effect against peripheral neuropathy induced by the anticancer drugs of interest herein by potentiating the effect of Nerve Growth Factor (NGF) (Cavaletti, G. et al., Journal of Neurology, Vol. 249, Suppl. 1, June 2002: 1/28, 78). NGF is extensively studied in the art as a neuroprotective agent in relation to Cisplatin and Taxol neurotoxicity. Acetyl L-carnitine is able to potentiate the effect of NGF on PC12 cells by enhancing expression of different genes, as assessed by microarray analysis, and a relationship between NGF and acetyl L-carnitine in promoting PC12 neuritis outgrowth was also reported. Another definite relationship between acetyl L-carnitine and NGF was found even in the animal model of chronic Cisplatin-induced sensory neuropathy. In this model, a close relationship has already been previously demonstrated between the onset of a sensory neuropathy and the decrease in NGF circulating levels.

A significantly reduced severity of neuropathy was also observed in the *in vivo* model of Taxol neurotoxicity in the Taxol-acetyl L-carnitine treated group. In addition to the effect in reducing the NGF fall in Cisplatin treated animals, acetyl L-carnitine seems to increase the cells response to NGF. Concerning the molecular mechanisms at the basis of the protective effect of acetyl L-carnitine, the studies performed in the present invention showed the ability of the protective agent to enhance neuronal NGF response via histone acetylation, which is involved in regulation of gene expression. The acetyl group of acetyl L-carnitine is transferred to histones in NGF-differentiated PC12 cells. The presence of acetyl L-carnitine increased NGF-induced histone acetylation. Moreover, the addition of acetyl L-carnitine to PC12 cells significantly stimulates the expression of NGFI-A, a gene coding for a transcription factor with tumor suppressor effects. In addition, NGFI-A protein is implicated in several physiological processes and it was suggested to have an important role in tissue repair. In conclusion, the present invention indicates that acetyl L-carnitine is a specific protective agent for chemotherapy-induced neuropathy following treatment with the anti-cancer agents of interest in the present invention. Relevant to this point is the lack of any interference with the antitumor activity of the drugs. Finally, acetyl L-carnitine can enhance the supportive effect of physiological NGF during chemotherapy-induced neuropathy, thus avoiding the problem of the local and general side effects of the exogenous administration of NGF which are a major problem of this neuroprotective strategy.

When ALC is used according to the invention described herein, no adverse effects on the thalidomide action of the drug are found.

ALC can be conveniently administered orally, without, for that reason, excluding other administration routes which an expert in the field may deem it advisable to adopt, particularly, by injection, to be administered concomitantly, for example, in the same infusion vial, with thalidomide or in sequence, as established by the expert in the field.

Since L-carnitine and other alkanoyl L-carnitines (herein collectively named as "a carnitine), in particular propionyl L-carnitine (PLC), showed beneficial effect with the therapeutic activity of anticancer agents, as disclosed in the aforementioned WO 00/06134, it can be advantageous to provide a combination, also in separate dosage forms, or in some way combined, of acetyl L-carnitine as a protective agent and one or more "carnitine" together with the anticancer agent according to the present invention. This combination can also comprise other anticancer agents which induce substantially reduced side effects, much particularly peripheral neuropathy, as a result of the combination according to the invention described herein. This latter embodiment is advantageous in case of polychemotherapy. It may also be advantageous to add L-carnitine to the above-mentioned combination.

It is therefore further evidently advantageous to provide a ternary combination, also in separate dosage forms, or in some way combined, of acetyl L-carnitine as a protective agent, propionyl L-carnitine as a synergistic agent and thalidomide according to the present invention. This combination also comprises other anticancer agents which show a synergistic effect or induce substantially reduced side effects as a result of the combination according to the invention described herein. This latter embodiment is advantageous in case of polychemotherapy. It may also be advantageous to add L-carnitine to the above-mentioned combination.

One specific object of the invention described herein is a pharmaceutical composition comprising a therapeutically effective amount of thalidomide together with a protective amount of acetyl L-carnitine and a synergistic amount of propionyl L-carnitine, in a mixture with pharmaceutically acceptable vehicles and/or excipients.

As regards those aspects relating to industrial applicability, the invention described herein also provides, in one of its possible embodiments, for a kit containing a) a pharmaceutical composition comprising a therapeutically effective amount of thalidomide; b) a pharmaceutical composition comprising acetyl L-carnitine or a pharmaceutically acceptable salt thereof in an amount suitable for producing a substantially protective action against the side effects on peripheral nervous system of said anticancer agent. The kit according to the invention described herein may also be presented in the form of a) a pharmaceutical composition comprising a therapeutically effective amount of thalidomide; b) a pharmaceutical composition comprising acetyl L-carnitine in an amount suitable for producing a substantially protective action against the side effects of said anticancer agent.

A guideline for the kits is found in the above mentioned WO 00/6134 in particular for the cases of combination with other carnitines.

It remains understood that the expert in the field may complete the experimental protocols with his or her own general knowledge of the field in which he or she operates, possibly resorting to neighbouring sectors in case of need.

We report here below the results of the experiments obtained by the combination of acetyl L-carnitine with the is mentioned in the reference examples anticancer agents. For experimental details, reference can be made to WO 00/6134 and the literature cited in this application.

### Reference Example

### Protective effect of acetyl L-carnitine on an experimental model of Oxaliplatin-induced peripheral neuropathy - Prevention treatment.

The purpose of this study is to demonstrate and evaluate the protective properties, by way of prevention, of acetyl L-carnitine administered one day prior to Oxaliplatin treatment for 4 weeks.

Peripheral neuropathy was induced by intraperitoneally administration of Oxaliplatin (2.7 mg/kg/1.5 ml) dissolved in distilled sterilized water, twice a week for 4 weeks (for a total of 8 administrations).

Acetyl L-carnitine was dissolved in distilled water and administered subcutaneously. The dose of 100 mg/1.5 ml/Kg/day was administered on the basis of individual body weight. ALC treatment occurred daily, starting a day before Oxaliplatin treatment, for 4 weeks. Control and Oxaliplatin groups were treated s.c. with vehicle.

Nerve conduction velocity (NCV) was measured in each animal under halotane anaesthesia, prior to start of treatment (basal), and the day after the last administration of Oxaliplatin (final).

NCV was determined with the following method: the animals were anaesthetised with a gaseous mixture composed of 0.45 halothane, nitrogen protoxide and oxygen. Nerve conduction velocity was measured in the tail, by placing stimulating electrodes at the base of the tail and a couple of recording ring electrodes distally in the tail 5 and 10 cm with respect to the stimulating electrodes.

The latencies of the potentials recorded at the two sites after nerve stimulation were determined (peak to peak) and the nerve conduction velocity was calculated accordingly.

The site of stimulations and recording were kept fixed on repeated examinations performed in each animal by marking the tail with permanent ink.

The recordings and stimulations of the tail nerve were done using an Ote Biomedica Phasis II electromyograph using a stimulation intensity equal to the threshold value with a duration of 100 microseconds. In view of reports in the literature of nerve conduction velocity depending on the animal body temperature, it was necessary to keep the latter constant throughout the experiment, measuring it with a rectal probe, with the aid of a BM 70002-type thermoregulator for animals (Biomedica Mangoni).

The velocity was measured in all groups of animals both in basal conditions and after 5 weeks of treatment.

The results were expressed as means ± standard deviation; significance was assessed using the "t"-test, for both independent and paired data, with a statistical significance cut-off of p<0.05.

The sensory nerve conduction velocity data measured on the caudal nerve are given in the Table 1 here below.

**Table 1**

| Oxaliplatin-induced neuropathy: sensory nerve conduction velocity (m/s) measured on the animals' tails in basal conditions and after treatment with acetyl L-carnitine. | | |
|---|---|---|
| TREATMENT | | |
| GROUP | BASAL | Oxaliplatin 8^{th} treatment |
| Control | 31.0±1.4 | 35.8± 1.7 |
| Oxaliplatin | 31.3±1.7 | 30.8± 1.2* |
| Acetyl L-carnitine + Oxaliplatin | 31.4±1.5 | 33.6±2.6 § |

| | | |
|---|---|---|
| Values are meant ± standard deviation. t-test (independent data) * =p<0.001 vs Control § =p<0.001 vs Oxaliplatin | | |

### Reference Example

### Protective effect of acetyl L-carnitine on an experimental model of Oxaliplatin-induced peripheral neuropathy - Therapeutic treatment.

The purpose of this study is to demonstrate and evaluate the protective properties, by way of therapy, of acetyl L-carnitine administered at the end of Oxaliplatin treatments, during a follow-up period of 3 weeks.

Peripheral neuropathy was induced by intraperitoneal administration of Oxaliplatin (3 mg/kg/1.5 ml) dissolved in distilled sterilized water, twice a week for 4 weeks (for a total of 8 administrations).

Acetyl L-carnitine was dissolved in distilled water and administered subcutaneously during the follow-up period. The dose of 100 mg/1.5 ml/Kg/day was administered on the basis of individual body weight. ALC treatment started the day after the last Oxaliplatin administration.

Control and Oxaliplatin groups were treated s.c. with vehicle.

Nerve conduction velocity (NCV) was measured in each animal under halotane anaesthesia, prior to start of treatment (baseline), the day after the last administration of Oxaliplatin (final) and weekly after a follow-up period of 3 weeks (recovery).

NCV was determined as in Example 1.

The sensory nerve conduction velocity data measured on the caudal nerve are given in the Table 2 here below.

**Table 2**

| Oxaliplatin-induced neuropathy: sensory nerve conduction velocity (m/s) measured on the animals' tail in basal conditions and after treatment with acetyl L-carnitine. | | | | | |
|---|---|---|---|---|---|
| TREATMENT | | | RECOVERY | | |
| GROUP | BASAL | 8th Oxaliplatin Treatment | 1st week | 2nd week | 3rd week |
| Control | 31.0±1.4 | 36.8± 1,7 *** | 37.8±2.3 *** | 38.2±1.5 *** | 39.3±1.2 *** |
| Oxaliplatin | 31.6±2.0 | 30.8± 0.6 *** | 31.6±2.7 *** | 32.0±1.9 ** | 35.9±1.8 * |
| Acetyl L-carnitine + Oxaliplatin | 31.0±1.7 | 30.6±1.5 | 34.1±2.6 § | 35.1±1.9 §§ | 36.6±2.0 |

| | | | | | |
|---|---|---|---|---|---|
| Values are means ± standard deviation. t-test (independent data) *=p<0.01 vs Control; **=p<0.005 vs Control; ***=p<0.0001 vs Control §=p<0.05 vs Oxaliplatin; §§=p<0.01 vs Oxaliplatin | | | | | |

### Reference Example

### Protective effect of acetyl L-carnitine on peripheral nerve from Oxaliplatin-induced damage - Therapeutic and preventive treatment.

The paw-withdrawal test (Randall-Sellitto) was used to address mechanical hyperalgesia. Nociceptive threshold was measured using an Ugo Basile analgesimeter by applying an increasing pressure to the left and right hind paws. The nocicpetive threshold is defined as the pressure (grams) at which the rat withdraws its paw. Lower thresholds correspond to an increased sensitivity to algesia. This behavioural test is conducted at different times (days) from drug or vehicle administration. Rats are made familiar with the testing procedure and with handling by investigators during the week prior to the experiments.

Wistar female rats (Charles River) about 10 weeks of age were used. Oxaliplatin (3 mg/kg i.p.) in saline was administered three times a week. For the first two weeks, then two times a week for two weeks and then once a week.

The preventive treatment was conducted as follows: Oxaliplatin 3 mg/kg i.p. + ALC 100 mg/Kg s.c., daily from 1st day of the Oxaliplatin treatment.

The therapeutic treatment was conducted as follows: Oxaliplatin 3 mg/kg i.p. + ALC 100 mg/Kg s.c., daily from 24th day of the Oxaliplatin treatment.

The nociceptive threshold data (Randall-Sellitto) are given in the Table 3 here below.

**Table 3**

| Oxaliplatin-induced neuropathy: nociceptive threshold (g) measured on the animals'hind paws (mean ± S.D.) | | | | | | | | | | |
|---|---|---|---|---|---|---|---|---|---|---|
| GROUP | N | Days | | | | | | | | |
| | | 0 | 9 | 16 | 23 | 30 | 37 | 40 | 44 | 51 |
| Saline | 9 | 235 | 245 | 214 | 204 | 234 | 215 | 234 | 204 | 209 |
| | | 48.6 | 83.8 | 38.4 | 41.5 | 59.5 | 37.2 | 65.3 | 39.4 | 46.0 |
| | | | d | a | d | d | | d | | |
| Saline + | 9 | 210 | 244 | 232 | 234 | 250 | 260 | 216 | 199 | 216 |
| ALC | | 37.4 | 99.1 | 65.5 | 77.9 | 96.9 | 100.0 | 79.6 | 81.8 | 82.7 |
| | | | d | d | d | d | a | a | | |
| Oxaliplatin | 15 | 217 | 129 | 133 | 121 | 114 | 151 | 143 | 147 | 136 |
| | | 41.2 | 35.7 | 41.2 | 42.2 | 59.1 | 94.3 | 46.4 | 107.1 | 60.8 |
| ALC + | 12 | 242 | 206 | 207 | 238 | 247 | 276 | 241 | 283 | 291 |
| Oxaliplatin | | 34.0 | 104.5 | 110.8 | 46.0 | 115.7 | 114.0 | 87.5 | 129.4 | 95.2 |
| Preventive | | | | a | d | d | d | d | d | d |
| ALC + | 14 | 221 | 181 | 136 | 116 | 182 | 179 | 191 | 186 | 196 |
| Oxaliplatin Therapeutic | | 32.3 | 92.2 | 44.0 | 42.1 | 76.9 | 50.2 | 46.3 | 68.5 | 98.1 |

| | | | | | | | | | | |
|---|---|---|---|---|---|---|---|---|---|---|
| ANOVA: Dunnett's multiple comparison test vs Oxaliplatin. a = p<0.05, d = p<0.001. | | | | | | | | | | |

The statistical analysis is given in Table 4 herein below.

**Table 4**

| | D.F. | F | P< |
|---|---|---|---|
| Saline vs Oxaliplatin (0-51 days) | | | |
| Treatment | 1-22 | 28.16 | 0.001 |
| Interaction | 8-176 | 2.03 | 0.045 |
| Oxaliplatin + ALC - Preventive | | | |
| Vs Oxaliplatin (0-51-days) | | | |
| Treatment | 1-25 | 21.98 | 0.001 |
| Interaction | 8-200 | 3.06 | 0.003 |
| Saline vs Oxaliplatin (23-51 days) | | | |
| Treatment | 1-22 | 17.95 | 0.001 |
| Interaction | 5-110 | 1.10 | NS |
| Oxaliplatin + ALC - Therapy | | | |
| Vs Oxaliplatin (23-51-days) | | | |
| Treatment | 1-27 | 6.03 | 0.021 |
| Interaction | 5-135 | 1.41 | NS |

### Reference Example

### Protective effect of acetyl L-carnitine on peripheral nerve from Vinorelbine-induced damage - Therapeutic and preventive treatment.

The paw-withdrawal test (Randall-Sellitto) was used as in Example 3. Sprague Dawley male rats (Harlan) about 3 months of age (about 350 g) were used. Vinorelbine (0.200 mg/kg i.p.) in saline was administered three times a week until the end of the experiment.

The preventive treatment was conducted as follows: Vinorelbine 3 mg/kg i.p. + ALC 100 mg/Kg s.c., daily (6 days a week) from 1^{st} day of the Vinorelbine treatment. Treatment with ALC went until day 25.

The therapeutic treatment was conducted as follows: Vinorelbin 3 mg/kg i.p. + ALC 100 mg/Kg s.c., daily (6 days a week) from 9^{th} day of the Vinorelbine treatment. Treatment with ALC went until day 25.

The test was done twice a week

The nociceptive threshold data (Randall-Sellitto) are given in the Table 5 here below.

**Table 5**

| Vinorelbine-induced neuropathy: nociceptive threshold (g) measured on the animals' hind paws (mean ± S.D.) | | | | | | | | | |
|---|---|---|---|---|---|---|---|---|---|
| GROUP | N | Days | | | | | | | |
| | | 0 | 4 | 9 | 11 | 16 | 18 | 23 | 25 |
| Saline | 10 | 228.5 | 214.6 | 229.8 | 242.9 | 220.7 | 205.0 | 196.8 | 214.8 |
| | | 44.1 | 59.2 | 54.1 | 35.5 | 38.6 | 38.9 | 46.9 | 54.5 |
| | | | | c | d | d | d | c | d |
| Vinorelbine | 12 | 211.4 | 173.5 | 168.8 | 156.8 | 135.8 | 127.7 | 136.2 | 122.7 |
| | | 32.9 | 51.6 | 45.6 | 50.5 | 36.0 | 38.2 | 45.4 | 40.9 |
| ALC + | 10 | 222.0 | 197.4 | 234.0 | 211.2 | 235.4 | 204.6 | 202.6 | 201.2 |
| Vinorelbine | | 23.8 | 39.6 | 74.5 | 41.4 | 44.4 | 35.6 | 41.8 | 36.1 |
| Preventive - 1 day | | | | a | b | d | d | c | d |
| ALC + | 12 | 216.5 | 167.5 | 160.2 | 190.6 | 196.2 | 227.2 | 209.0 | 206.7 |
| Vinorelbine | | 44.3 | 41.6 | 60.6 | 70.2 | 49.5 | 72.6 | 56.9 | 59.8 |
| Therapeutic - 9 days | | | | | | b | d | c | d |

| | | | | | | | | | |
|---|---|---|---|---|---|---|---|---|---|
| Student's "t" test vs Vinorelbine a = p≤0.05, b = p≤0.02, c = p≤0.01 d = p≤0.001. | | | | | | | | | |

### Reference Example

### Protective effect of acetyl L-carnitine on peripheral nerve from Vincristine-induced damage - Therapeutic and preventive treatment.

The paw-withdrawal test (Randall-Sellitto) was used as in Example 3.

Sprague Dawley male rats (Harlan) about 3 months of age (about 350 g) were used. Vincristine (0.150 mg/kg i.p.) in saline was administered three times a week until the end of the experiment.

The preventive treatment was conducted as follows: Vincristine 0.150 mg/kg i.p. three times a week + ALC 100 mg/Kg s.c., daily (6 days a week).

The therapeutic treatment was conducted as follows: Vincristine 0.150 mg/kg i.p. + ALC 100 mg/Kg s.c., daily (6 days a week) from 15th day of the Vincristine treatment.

The test was done twice a week.

The nociceptive threshold data (Randall-Sellitto) are given in the Table 6 and Table 7 here below.

**Table 6**

| Vincristine-induced neuropathy: nociceptive threshold (g) measured on the animals' hind paws (mean ± S.D.) - preventive treatment | | | | | | | | |
|---|---|---|---|---|---|---|---|---|
| GROUP | N° | Days | | | | | | |
| | | 0 | 3 | 8 | 10 | 15 | 17 | 22 |
| | | 212.7 | 220.0 | 202.0 | 221.3. | 213.3 | 213.3 | 222.0 |
| Saline | 30 | 50.4 | 67.4 | 59.2 | 63.5 | 60.2 | 72.3 | 73.9 |
| | | | d | d | d | d | d | d |
| Vincristine | 30 | 222.0 | 137.3 | 114.7 | 117.3 | 126.7 | 94.7 | 133.0 |
| | | 43.8 | 52.6 | 53.1 | 48.8 | 69.6 | 80.5 | 73.9 |
| ALC + Vincristine | 30 | 210.7 | 206.7 | 209.3 | 213.3 | 213.3 | 202.7 | 215.0 |
| | | 41.1 | 66.8 | 81.1 | 83.8 | 59.2 | 64.6 | 72.8 |
| | | | d | d | d | d | d | d |

| | | | | | | | | |
|---|---|---|---|---|---|---|---|---|
| Student's "t" test vs Vincristine d = p<0.001. | | | | | | | | |

**Table 7**

| Vincristine-induced neuropathy: nociceptive threshold (g) measured on the animals' hind paws (mean ± S.D.) - therapeutic treatment | | | | | | | | | | | | |
|---|---|---|---|---|---|---|---|---|---|---|---|---|
| GROUP | N° | Days | | | | | | | | | | |
| | | 0 | 4 | 8 | 10 | 15 | 22 | 24 | 29 | 31 | 36 | 38 |
| | | | 233.5 | 201.5 | 209.4 | 224.8 | 231.0 | 223.5 | 215.9 | 222.2 | 250.4 | 248.6 |
| | | 197.2 | | | | | | | | | | |
| Saline | 10 | | 60.7 | 55.7 | 47.4 | 59.5 | 47.4 | 62.6 | 44.9 | 46.2 | 86.3 | 62.0 |
| | | 24.0 | | | | | | | | | | |
| | | | a | | d | d | d | c | c | d | c | c |
| | | 199.4 | 173.6 | 146.6 | 131.4 | 127.4 | 120.6 | 133.0 | 128.0 | 129.2 | 137.8 | 154.2 |
| Vincristine | 10 | | | | | | | | | | | |
| | | 25.0 | 64.8 | 71.8 | 31.9 | 50.9 | 40.2 | 74.6 | 56.9 | 46.2 | 52.8 | 64.2 |
| | | | | | | | 188.6 | 230.5 | 248.4 | 282.0 | 265.0 | 281.6 |
| ALC + | | 216.2 | 212.8 | 186.8 | 152.6 | 128.0 | | | | | | |
| | 10 | | | | | | 72.4 | 68.9 | 100.2 | 76.8 | 71.5 | 97.1 |
| Vincristine | | 38.9 | 62.9 | 87.3 | 59.5 | 39.8 | | | | | | |
| | | | | | | | b | c | c | d | d | c |

| | | | | | | | | | | | | |
|---|---|---|---|---|---|---|---|---|---|---|---|---|
| Student's "t" test vs Vincristine b = p≤0.02, c = p≤0.01 d = p≤0.001. | | | | | | | | | | | | |

## Claims

1. The use of acetyl L-carnitine or of a pharmaceutically acceptable salt thereof for the preparation of a medicament for preventing and/or treating peripheral neuropathies induced by the administration of thalidomide.

2. The use according to claim 1, wherein said medicament is suitable for the administration in a co-ordinated manner.

3. The use according to claim 2, wherein said administration is substantially simultaneous.

4. The use according to claim 2, wherein said administration is sequential.

5. The use according to any of claims 2-4, wherein said administration is in the form of a composition comprising said acetyl L-carnitine or a pharmaceutically acceptable salt thereof in combination and in a mixture with said anticancer agent in addition to optional pharmaceutically acceptable excipients and/or vehicles.

6. The use according to any of the preceding claims, wherein 0.1 to 3 g/day of acetyl L-carnitine or of an equivalent amount of a pharmaceutically acceptable salt thereof are administered.

7. The use according to any one of the preceding claims, wherein said pharmacologically acceptable salt of acetyl L-carnitine is selected from the group consisting of chloride, bromide, orotate, acid aspartate, acid citrate, acid phosphate, fumarate and acid fumarate, maleate and acid maleate, acid oxalate, acid sulphate, glucose phosphate, tartrate and acid tartrate.

8. The use according to any one of claims 1-2 and 4-7, wherein said medicament is to be administered to a subject in view of the need of a treatment with said anticancer agent.

9. The use according to claim 8, wherein said medicament is to be administered immediately before or immediately after surgical removal of the tumor.

10. The use according to claim 8, wherein said anticancer agent is alternative to surgical removal of the tumor.

11. The use according to any of the preceding claims, wherein a further carnitine or a pharmaceutically acceptable salt thereof is added.

12. The use according to any of the preceding claims, wherein a further anticancer agent is added.

13. A composition which in combination comprises:
a) acetyl L-carnitine or a pharmaceutically acceptable salt thereof;
b) thalidomide.

14. The composition according to claim 13, wherein said pharmacologically acceptable salt of acetyl L-carnitine is selected from the group consisting of chloride, bromide, orotate, acid aspartate, acid citrate, acid phosphate, fumarate and acid fumarate, maleate and acid maleate, acid oxalate, acid sulphate, glucose phosphate, tartrate and acid tartrate.

15. The composition according to any of Claims 13-14, wherein a further carnitine or a pharmaceutically acceptable salt thereof is added.

16. The composition according to any of Claims 13-15, wherein a further anticancer agent is added.

17. A kit comprising a) a pharmaceutical composition comprising a therapeutically effective amount of thalidomide; b) a pharmaceutical composition comprising acetyl L-carnitine or a pharmaceutically acceptable salt thereof in an amount suitable for producing a substantially protective action against peripheral neuropathies induced by the administration of thalidomide.

18. The kit according to claim 17, wherein said pharmacologically acceptable salt of acetyl L-carnitine is selected from the group consisting of chloride, bromide, orotate, acid aspartate, acid citrate, acid phosphate, fumarate and acid fumarate, maleate and acid maleate, acid oxalate, acid sulphate, glucose phosphate, tartrate and acid tartrate.

19. The kit according to any of claims 17-18, wherein a further carnitine or a pharmaceutically acceptable salt thereof is added.

20. The kit according to any of claims 17-19, wherein a further anticancer agent is added.

## Patentansprüche

1. Verwendung von Acetyl-L-carnitin oder eines pharmazeutisch verträglichen Salzes davon, für die Herstellung eines Medikaments zur Prävention und/oder Behandlung peripherer Neuropathien, welche durch die Verabreichung von Thalidomid induziert werden.

2. Verwendung gemäß Anspruch 1, worin das genannte Medikament zur Verabreichung in koordinierter Weise geeignet ist.

3. Verwendung gemäß Anspruch 2, worin die genannte Verabreichung im Wesentlichen simultan ist.

4. Verwendung gemäß Anspruch 2, worin die genannte Verabreichung sequenziell ist.

5. Verwendung gemäß irgendeinem der Ansprüche 2 - 4, worin die genannte Verabreichung die Form einer Zusammensetzung hat, umfassend das genannte Acetyl-L-carnitin oder ein pharmazeutisch verträgliches Salz davon, in Kombination und in einer Mischung mit dem genannten Antikrebsmittel zusätzlich zu optionalen Hilfsstoffen und/oder Trägerstoffen.

6. Verwendung gemäß irgendeinem der vorangehenden Ansprüche, worin 0,1 bis 3 g/Tag Acetyl-L-carnitin oder eine äquivalente Menge eines pharmazeutisch verträglichen Salzes davon verabreicht werden.

7. Verwendung gemäß irgendeinem der vorangehenden Ansprüche, worin das pharmakologisch verträgliche Salz von Acetyl-L-carnitin ausgewählt ist aus der Gruppe, bestehend aus Chlorid, Bromid, Orotat, saurem Aspartat, saurem Citrat, saurem Phosphat, Fumarat und saurem Fumarat, Maleat und saurem Maleat, saurem Oxalat, saurem Sulfat, Glucosephosphat, Tartrat und saurem Tartrat.

8. Verwendung gemäß irgendeinem der Ansprüche 1 - 2 und 4 - 7, worin das genannte Medikament einem Subjekt angesichts des Bedarfs einer Behandlung mit dem genannten Antikrebsmittel verabreicht wird.

9. Verwendung gemäß Anspruch 8, worin das genannte Medikament direkt vor oder sofort nach der operativen Entfernung des Tumors verabreicht wir.

10. Verwendung gemäß Anspruch 8, worin das genannte Antikrebsmittel eine Alternative zur operativen Entfernung des Tumors ist.

11. Verwendung gemäß irgendeinem der vorangehenden Ansprüche, worin ein weiteres Carnitin oder ein pharmazeutisch verträgliches Salz davon hinzugefügt wird.

12. Verwendung gemäß irgendeinem der vorangehenden Ansprüche, worin ein weiteres Antikrebsmittel hinzugefügt wird.

13. Zusammensetzung, welche als Kombination umfasst:
a) Acetyl-L-carnitin oder ein pharmazeutisch verträgliches Salz davon;
b) Thalidomid.

14. Zusammensetzung gemäß Anspruch 13, worin das genannte pharmakologisch verträgliche Salz von Acetyl-L-carnitin ausgewählt ist aus der Gruppe, bestehend aus Chlorid, Bromid, Orotat, saurem Aspartat, saurem Citrat, saurem Phosphat, Fumarat und saurem Fumarat, Maleat und saurem Maleat, saurem Oxalat, saurem Sulfat, Glucosephosphat, Tartrat und saurem Tartrat.

15. Zusammensetzung gemäß irgendeinem der Ansprüche 13 - 14, worin ein weiteres Carnitin oder ein pharmazeutisch verträgliches Salz davon hinzugefügt wird.

16. Zusammensetzung gemäß irgendeinem der Ansprüche 13 - 15, worin ein weiteres Antikrebsmittel hinzugefügt wird.

17. Kit, umfassend a) eine pharmazeutische Zusammensetzung, umfassend eine therapeutisch wirksame Menge Thalidomid; b) eine pharmazeutische Zusammensetzung, umfassend Acetyl-L-carnitin oder ein pharmazeutisch verträgliches Salz davon, in einer Menge, die geeignet ist, eine wesentliche Schutzwirkung gegen periphere Neuropathien zu erzeugen, welche durch die Verabreichung von Thalidomid induziert werden.

18. Kit gemäß Anspruch 17, worin das genannte pharmakologisch verträgliche Salz von Acetyl-L-carnitin ausgewählt ist aus der Gruppe, bestehend aus Chlorid, Bromid, Orotat, saurem Aspartat, saurem Citrat, saurem Phosphat, Fumarat und saurem Fumarat, Maleat und saurem Maleat, saurem Oxalat, saurem Sulfat, Glucosephosphat, Tartrat und saurem Tartrat.

19. Kit gemäß irgendeinem der Ansprüche 17 - 18, worin ein weiteres Carnitin oder ein pharmazeutisch verträgliches Salz davon hinzugefügt wird.

20. Kit gemäß irgendeinem der Ansprüche 17 - 18, worin ein weiteres Antikrebsmittel hinzugefügt wird.

## Revendications

1. Utilisation d'acétyl-L-carnitine ou d'un sel pharmaceutiquement acceptable de celle-ci pour la préparation d'un médicament destiné à prévenir et/ou traiter les neuropathies périphériques induites par l'administration de thalidomide.

2. Utilisation selon la revendication 1, dans laquelle ledit médicament est approprié pour l'administration d'une manière coordonnée.

3. Utilisation selon la revendication 2, dans laquelle ladite administration est sensiblement simultanée.

4. Utilisation selon la revendication 2, dans laquelle ladite administration est séquentielle.

5. Utilisation selon l'une quelconque des revendications 2 à 4, dans laquelle ladite administration est sous la forme d'une composition comprenant ladite acétyl-L-carnitine ou un sel pharmaceutiquement acceptable de celle-ci en combinaison et en mélange avec ledit agent anticancéreux en plus d'excipients et/ou véhicules optionnels pharmaceutiquement acceptables.

6. Utilisation selon l'une quelconque des revendications précédentes, dans laquelle 0,1 à 3 g/jour d'acétyl-L-carnitine ou une quantité équivalente d'un sel pharmaceutiquement acceptable de celle-ci sont administrés.

7. Utilisation selon l'une quelconque des revendications précédentes, dans laquelle ledit sel pharmaceutiquement acceptable d'acétyl-L-carnitine est choisi dans le groupe constitué du chlorure, bromure, orotate, aspartate acide, citrate acide, phosphate acide, fumarate et fumarate acide, maléate et maléate acide, oxalate acide, sulfate acide, phosphate de glucose, tartrate et tartrate acide.

8. Utilisation selon l'une quelconque des revendications 1 à 2 et 4 à 7, dans laquelle ledit médicament est destiné à être administré à un sujet en raison du besoin d'un traitement avec ledit agent anticancéreux.

9. Utilisation selon la revendication 8, dans laquelle ledit médicament doit être administré immédiatement avant ou immédiatement après une ablation chirurgicale de la tumeur.

10. Utilisation selon la revendication 8, dans laquelle ledit agent anticancéreux est une alternative à l'ablation chirurgicale de la tumeur.

11. Utilisation selon l'une quelconque des revendications précédentes, dans laquelle une autre carnitine ou un sel pharmaceutiquement acceptable de celle-ci est ajouté.

12. Utilisation selon l'une quelconque des revendications précédentes, dans laquelle un autre agent anticancéreux est ajouté.

13. Composition qui comprend en combinaison :
a) acétyl-L-carnitine ou un sel pharmaceutiquement acceptable de celle-ci ;
b) thalidomide.

14. Composition selon la revendication 13, dans laquelle ledit sel pharmaceutiquement acceptable d'acétyl-L-carnitine est choisi dans le groupe constitué du chlorure, bromure, orotate, aspartate acide, citrate acide, phosphate acide, fumarate et fumarate acide, maléate et maléate acide, oxalate acide, sulfate acide, phosphate de glucose, tartrate et tartrate acide.

15. Composition selon l'une quelconque des revendications 13 et 14, dans laquelle une autre carnitine ou un sel pharmaceutiquement acceptable de celle-ci est ajouté.

16. Composition selon l'une quelconque des revendications 13 à 15, dans laquelle un autre agent anticancéreux est ajouté.

17. Ensemble de conditionnement comprenant a) une composition pharmaceutique comprenant une quantité pharmaceutiquement efficace de thalidomide ; b) une composition pharmaceutique comprenant de l'acétyl-L-carnitine ou un sel pharmaceutiquement acceptable de celle-ci en une quantité appropriée pour produire une action sensiblement protectrice contre des neuropathies périphériques induites par l'administration de thalidomide.

18. Ensemble de conditionnement selon la revendication 17, dans lequel ledit sel pharmacologiquement acceptable d'acétyl-L-carnitine est choisi dans le groupe constitué du chlorure, bromure, orotate, aspartate acide, citrate acide, phosphate acide, fumarate et fumarate acide, maléate et maléate acide, oxalate acide, sulfate acide, phosphate de glucose, tartrate et tartrate acide.

19. Ensemble de conditionnement selon l'une quelconque des revendications 17 à 18, dans lequel une autre carnitine ou un sel pharmaceutiquement acceptable de celle-ci est ajoutée.

20. Ensemble de conditionnement selon l'une quelconque des revendications 17 à 19, dans lequel un autre agent anticancéreux est ajouté.
